(19) Europäisches Patentamt

European Patent Office

Office européen des brevets

(11) **EP 1 409 079 B1**

(12) **FASCICULE DE BREVET EUROPEEN**

(45) Date de publication et mention
de la délivrance du brevet:
**29.03.2006 Bulletin 2006/13**

(21) Numéro de dépôt: **02764948.2**

(22) Date de dépôt: **05.07.2002**

(51) Int Cl.:
***A61N 7/02*** (2006.01)

(86) Numéro de dépôt international:
**PCT/FR2002/002367**

(87) Numéro de publication internationale:
**WO 2003/008041 (30.01.2003 Gazette 2003/05)**

(54) **SONDE DE TRAITEMENT PAR ULTRASONS FOCALISES**

THERAPEUTISCHE ULTRASCHALLSONDE

TREATMENT PROBE FOR FOCUSSED ULTRASOUND

(84) Etats contractants désignés:
**AT BE BG CH CY CZ DE DK EE ES FI FR GB GR
IE IT LI LU MC NL PT SE SK TR**

(30) Priorité: **13.07.2001 FR 0109370**

(43) Date de publication de la demande:
**21.04.2004 Bulletin 2004/17**

(73) Titulaire: **TECHNOMED MEDICAL SYSTEMS
69120 Vaulx-en-Velin (FR)**

(72) Inventeurs:
• **LACOSTE, François**
**F-92500 Rueil Malmaison (FR)**

• **ESNAULT, Olivier**
**F-75017 Paris (FR)**

(74) Mandataire: **Hirsch & Associés
58, avenue Marceau
75008 Paris (FR)**

(56) Documents cités:
**WO-A-89/07909          DE-A- 4 404 140
FR-A- 2 750 340          US-A- 4 938 216
US-A- 5 178 135          US-A- 5 273 027
US-A- 6 036 661**

**Description**

La présente invention concerne le domaine des ultrasons focalisés, et plus précisément le traitement par ultrasons focalisés.

Les ultrasons focalisés permettent de traiter des tissus profonds sans accès direct à ces tissus. Un faisceau ultrasonore focalisé provenant d'un transducteur de puissance est concentré vers un foyer, qui est positionné sur la cible. Il en résulte un double phénomène thermique et de cavitation. L'effet tissulaire dépend de l'application de l'énergie ultrasonore. Sous certaines conditions (intensité acoustique modérée), on obtient un effet thermique, sous d'autres (intensité acoustique forte), l'effet de cavitation est prépondérant. Le choix des paramètres de traitement (intensité et fréquence acoustique, durée des tirs, durée des pauses entre les tirs, espacement entre les tirs, ...) s'effectue de sorte à éviter les brûlures dans les tissus intermédiaires, c'est à dire situés entre la source ultrasonore et la cible. On appelle "axe acoustique" du transducteur une ligne joignant le centre du transducteur, ou son centre de symétrie (s'il existe) et le foyer. Dans le cas d'un transducteur plan et d'une focalisation électronique, l'axe acoustique est l'axe perpendiculaire au plan du transducteur, et passant par le foyer; on peut aussi définir généralement l'axe acoustique comme passant par le foyer et dirigé suivant la direction moyenne de propagation des ultrasons.

L'effet de chaque impulsion ultrasonore est généralement limité à une zone spatiale de petite taille, dans laquelle l'intensité du champ ultrasonore est la plus forte, et qui est située autour du foyer. La zone focale aura typiquement la forme d'un cylindre de diamètre 1,5 mm dans un plan perpendiculaire à la direction de propagation des acoustique et de longueur 10 mm dans la direction de propagation acoustique.

Cette technique est particulièrement intéressante lorsque le traitement doit être précis, par exemple lorsque la zone à traiter est proche d'organes sensibles à préserver. C'est le cas par exemple du traitement de la prostate dans laquelle le sphincter externe ne doit pas être touché sous peine de provoquer l'incontinence du patient.

Il a donc été proposé d'associer dans un appareil de thérapie un transducteur de traitement et un transducteur d'imagerie ; en effet, le repérage ultrasonore est intéressant parce qu'il est simple, peu coûteux et n'émet pas de rayonnement ionisant. Le transducteur d'imagerie est utilisé, comme son nom l'indique, pour obtenir une image de la zone à traiter. Le transducteur de traitement, ou transducteur de puissance est utilisé pour l'émission des ultrasons destinés au traitement. Du point de vue quantitatif, la gamme de puissances moyennes pour le transducteur d'imagerie est typiquement de l'ordre de 0,1 à 1 W , tandis que la gamme de puissances moyennes pour le transducteur de thérapie est typiquement de l'ordre de 5 à 100 W. De plus, les impulsions ultrasonores émises pour l'imagerie ont une durée typique de 0,1 $\mu$s à 1 $\mu$s, tandis que les impulsions de thérapie durent de 0,1 s à 20 s. Pour permettre de visualiser un volume contenant la cible, un déplacement du plan de balayage du transducteur d'imagerie peut être prévu.

Des appareils de thérapie à ultrasons associés à un repérage échographique ont été décrits. Dans EP-A-0 148 653, EP-A-0 162 735 et US-A-5 431 621, un transducteur d'imagerie est logé au centre d'une coupelle servant de transducteur de traitement; cette coupelle présente une symétrie axiale. Le plan de balayage du transducteur d'imagerie contient l'axe acoustique du transducteur de traitement. Le transducteur échographique d'imagerie peut tourner sur son axe, mais ce n'est pas le cas du transducteur de traitement. Il est proposé dans ces documents d'utiliser l'appareil pour la destruction des calculs rénaux par ondes de choc ou pour le traitement de tumeurs par hyperthermie.

WO-A-92 15253 décrit une sonde endorectale présentant un transducteur à pans coupés. La sonde est montée à rotation sur un support et en translation suivant son axe longitudinal. Le transducteur de traitement est fixe par rapport au corps de la sonde. La sonde présente un transducteur d'imagerie, qui est fixe ou mobile par rapport au transducteur de traitement. Dans tous les cas, 1e plan de balayage du transducteur d'imagerie contient le foyer du transducteur de traitement.

EP-A-0 714 266 décrit une sonde endorectale adaptée au traitement de la prostate. La sonde comporte un transducteur de thérapie et un transducteur d'imagerie escamotables. En position « imagerie », le second transducteur balaye un plan contenant l'axe acoustique de traitement. Le plan de balayage est variable, puisqu'il peut pivoter autour de cet axe. Le transducteur de traitement ne tourne pas autour de son axe acoustique, mais autour d'un axe qui est parallèle à l'axe de la sonde endorectale.

WO-A-89 07909 propose, à la figure 2, un appareil de traitement extracorporel comportant un transducteur d'imagerie et un transducteur de traitement. Chacun des transducteurs est monté à l'extrémité d'un tube; les deux tubes sont montés sur un disque et s'étendent perpendiculairement au plan du disque. Le disque est monté à rotation dans l'appareil. Le tube portant le transducteur de traitement est sensiblement au centre du disque; ce tube est mobile en translation le long de son axe. A l'extrémité du tube, le transducteur de traitement est monté à rotation sur un axe perpendiculaire à l'axe du tube. Le transducteur de traitement dispose ainsi de trois degrés de liberté, pour être orienté dans toutes les directions. Le transducteur d'imagerie est monté de façon analogue; dans tous les cas, le plan de balayage du transducteur d'imagerie contient le foyer du transducteur de traitement. L'axe de rotation du disque - qui est l'axe longitudinal du tube portant le transducteur de traitement - ne correspond en général ni à l'axe acoustique du transducteur de traitement, ni à celui du transducteur d'imagerie: en effet, pour une profondeur de traitement donnée, le mouvement de balayage de la cible par le point focal s'effectue par rotation du transducteur de traitement autour de l'axe perpendiculaire au tube.

WO-A-95 02994 propose, à la figure 5, une sonde adaptée à visualiser et à traiter des tissus situés dans l'axe longitudinal de la sonde, tels que les tumeurs du foie ou les fibromes. Cette sonde présente un transducteur d'imagerie et un transducteur de thérapie montés dos à dos, l'ensemble étant monté à rotation à l'extrémité de la sonde, autour d'un axe perpendiculaire à l'axe de la sonde. La rotation du corps de la sonde permet de modifier le plan de balayage de la sonde. La rotation des transducteurs assure un balayage ou un traitement dans le plan concerné. Comme dans le document précédent, l'axe de rotation du corps de sonde - qui est l'axe longitudinal de la sonde - ne correspond en général pas à l'axe acoustique du transducteur de traitement. EP-A-0 273 180 propose une sonde du même type.

Ces différents appareils de l'état de la technique ne sont peu ou pas adaptés au traitement d'organes depuis l'extérieur du corps, et par exemple au traitement par ultrasons focalisés de la thyroïde. Il existe donc un besoin d'un appareil qui puisse traiter des organes tels que la thyroïde, par ultrasons focalisés, simplement, avec précision et efficacité.

Dans un mode de réalisation, l'invention telle que définie dans la revendication 1 propose une sonde de traitement par ultrasons focalisés, qui est adaptée au traitement de différents organes, depuis l'extérieur du corps. La sonde présente un corps de sonde, qui est montée à rotation sur un support. Le corps de sonde comporte un transducteur de traitement de forme allongée, dont l'axe acoustique est sensiblement confondu avec l'axe de rotation du corps de sonde. Le corps de sonde présente aussi un transducteur d'imagerie, dont le plan de balayage contient l'axe acoustique du transducteur de traitement.

Le fait que le transducteur soit allongé permet une bonne précision d'émission : le cône formé par les ondes acoustiques est dissymétrique, et présente un angle au sommet - au foyer - moins ouvert dans la direction transversale du transducteur que dans la direction longitudinale du transducteur. Il est plus facile d'éviter les organes proches de la cible. Le fait que l'axe acoustique soit sensiblement confondu avec l'axe de rotation du corps de sonde assure que le corps de sonde peut tourner autour de l'axe de rotation, sans pour autant que la position du foyer par rapport à la cible ne bouge. Il est alors possible de déplacer le corps de sonde en rotation, pour l'imagerie ou le traitement, sans pour autant déplacer les transducteurs.

Comme le plan de balayage - ou plan d'imagerie - du transducteur d'imagerie contient l'axe acoustique et contient donc aussi l'axe de rotation du corps de sonde, la cible se trouvant au foyer du transducteur de traitement reste dans le plan d'imagerie lors de la rotation du corps de sonde.

L'invention propose en outre que le transducteur de traitement soit monté mobile dans le corps de sonde. Il est notamment possible que le transducteur de traitement soit monté à rotation dans le corps de sonde autour d'un axe perpendiculaire à l'axe de rotation.

Cette configuration permet que le transducteur d'imagerie soit aussi monté mobile dans le corps de sonde. Il peut notamment être monté en translation dans le corps de sonde, de préférence suivant une direction parallèle à l'axe de rotation.

Dans un mode de réalisation, le transducteur de traitement présente un allongement supérieur à 1,2. Il est aussi avantageux que le transducteur de traitement présente un allongement inférieur à 2,5.

De préférence, le transducteur d'imagerie est une barrette effectuant un balayage linéaire. Il est alors avantageux que l'axe de ce transducteur soit parallèle à la direction longitudinale du transducteur de traitement. On peut notamment intégrer le transducteur d'imagerie au transducteur de traitement.

Dans un autre mode de réalisation, la sonde présente un support sur lequel la sonde est montée à rotation, le support déplaçant la sonde en translation dans un plan perpendiculaire à l'axe de rotation. Dans ce cas, le support peut en outre déplacer la sonde en translation suivant la direction de l'axe de rotation.

Le support peut aussi présenter une rotule d'orientation de la sonde. Une autre solution est que le support présente un arceau le long duquel la sonde se déplace ; il est alors avantageux que le rayon de l'arceau soit sensiblement égal à la distance entre l'arceau et le foyer du transducteur de traitement.

L'invention a donc pour objet une sonde de traitement par ultrasons focalisés, comportant un corps de sonde monté à rotation autour d'un axe, un transducteur de traitement de forme allongée, avec un axe acoustique d'émission d'ultrasons focalisés sensiblement confondu avec l'axe de rotation du corps de sonde, un transducteur d'imagerie dont le plan d'imagerie contient l'axe acoustique du transducteur de traitement.

Selon une variante, le transducteur de traitement est monté mobile dans le corps de sonde.

Selon une autre variante, le transducteur de traitement est monté à rotation dans le corps de sonde, de préférence autour d'un axe perpendiculaire à l'axe de rotation.

Selon encore une variante, le transducteur d'imagerie est monté mobile dans le corps de sonde.

Selon encore une autre variante, le transducteur d'imagerie est monté en translation dans le corps de sonde, de préférence suivant une direction parallèle à l'axe de rotation.

On peut également prévoir que le transducteur de traitement présente un allongement supérieur à 1,2. On peut prévoir avantageusement que le transducteur de traitement présente un allongement inférieur à 2,5.

Selon une variante, le transducteur d'imagerie est une barrette effectuant un balayage linéaire.

Selon une autre variante, l'axe du transducteur d'imagerie est parallèle à la direction longitudinale du transducteur de traitement.

Selon encore une variante, le transducteur d'imagerie est intégré au transducteur de traitement.

Selon encore une autre variante, la sonde présente un support sur lequel la sonde est montée à rotation, le support déplaçant la sonde en translation dans un plan perpendiculaire à l'axe de rotation.

On peut en outre prévoir que le support déplace en outre la sonde en translation suivant la direction de l'axe de rotation.

Selon une variante, le support présente une rotule d'orientation de la sonde.

Selon encore une variante, le support présente un arceau le long duquel la sonde se déplace.

Selon une autre variante, le rayon de l'arceau est sensiblement égal à la distance entre l'arceau et le foyer du transducteur de traitement.

D'autres caractéristiques et avantages de l'invention apparaîtront à la lecture de la description qui suit de modes de réalisation de l'invention, donnée à titre d'exemple et en référence aux dessins annexés qui montrent :

- figure 1, une vue schématique en coupe longitudinale d'une sonde, suivant la direction longitudinale du transducteur de traitement ;
- figure 2, une vue schématique en coupe longitudinale de la sonde de la figure 1, suivant la direction transversale du transducteur de traitement ;
- figure 3 une vue schématique de face de la sonde des figures 1 et 2 ;
- figure 4, une vue schématique de la sonde des figures 1 et 2, dans une application au traitement de la thyroïde ;
- figures 5, 6 et 7, des vues schématiques correspondant aux figures 1, 2 et 3, lorsque la sonde est en position d'imagerie ;
- figure 8, une vue schématique partielle d'un autre exemple de sonde, en coupe longitudinale suivant la direction longitudinale du transducteur de traitement ;
- figure 9, une vue schématique partielle de face de la sonde de la figure 8 ;
- figures 10 et 11, des vues de la position du corps de sonde par rapport à un patient, dans une application au traitement de la thyroïde ;
- figure 12, une vue schématique d'un exemple de sonde, montrant le support de sonde ;
- figure 13, une vue analogue à celle de la figure 12, pour un autre exemple de support de sonde ;
- figure 14, un ordinogramme d'un traitement à l'aide de la sonde ;
- figure 15, une vue du transducteur de traitement expliquant les paramètres de tir.

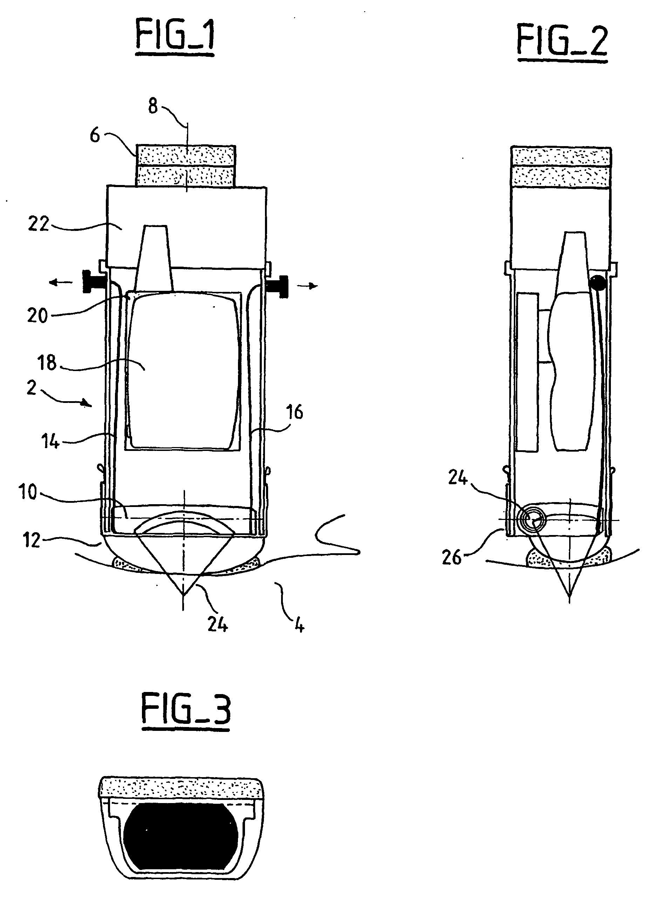

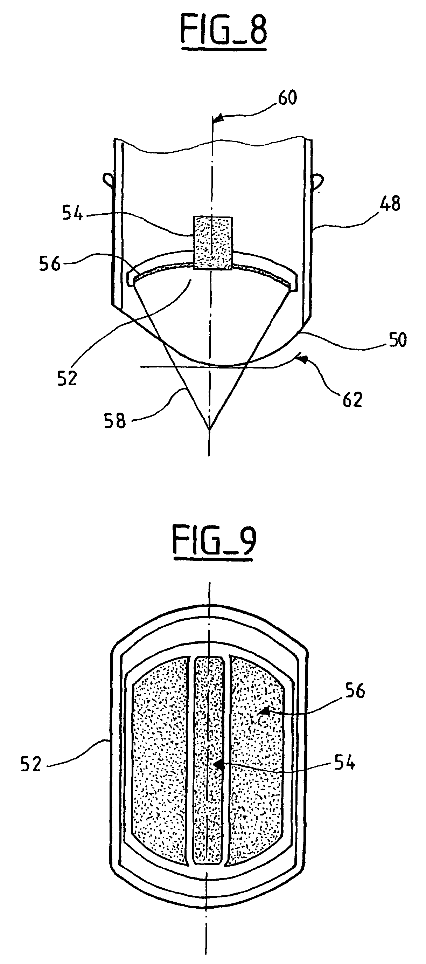

Les figures 1 à 7 montrent un exemple dans lequel le transducteur de traitement et le transducteur d'imagerie sont mobiles dans le corps de sonde; à l'inverse, les figures 8 et 9 montrent un exemple dans lequel le transducteur de traitement et le transducteur d'imagerie sont fixes dans le corps de sonde.

La figure 1 est une vue schématique en coupe longitudinale d'une sonde, suivant la direction longitudinale du transducteur de traitement. La coupe longitudinale de la sonde s'effectue dans un plan contenant l'axe de rotation du corps de sonde; il s'avère dans l'exemple de la figure 1 que le corps de sonde présente une forme allongée et que l'axe longitudinal du corps de sonde est confondu avec l'axe de rotation. La direction longitudinale du transducteur est définie, du fait de la forme allongée du transducteur de traitement, comme la direction suivant laquelle le transducteur de traitement présente la dimension la plus importante; la direction transversale du transducteur de traitement est à l'inverse la direction perpendiculaire à l'axe acoustique suivant laquelle la dimension du transducteur de traitement est la plus réduite.

La figure 1 montre le corps de sonde 2, ainsi qu'une partie du patient 4 en cours de traitement. Le corps de sonde est monté à rotation sur un support de sonde (non représenté), par une liaison 6 ; l'axe 8 est l'axe de rotation du corps de sonde sur le support de sonde. Dans sa partie frontale, qui est dirigée vers la zone à traiter du patient, le corps de sonde présente le transducteur de traitement 10. Dans l'exemple de la figure, il s'agit d'un transducteur à pan coupés, comme le montre 1a figure 3. Le transducteur de traitement présente dans l'exemple une focalisation naturelle, du fait de sa forme; il pourrait aussi être formé d'une pluralité de transducteurs indépendamment excitables, et présenter une focalisation électronique. L'extrémité du corps de sonde est formée par un ballonnet 12, souple et transparent aux ultrasons. Ce ballonnet est gonflé par un liquide de couplage transparent aux ultrasons, à l'aide de conduites de liquide 14 et 16. Les conduites sont fixées sur le corps de sonde afin de permettre un nettoyage aisé, ou pouvoir être changées facilement; ces conduites se terminent par des connecteurs pour l'injection, l'extraction ou la circulation du liquide de couplage; ces conduites débouchent avantageusement au voisinage du ballonnet. On peut utiliser le liquide de couplage décrit dans FR 99 03738, qui est refroidi pour protéger le transducteur de traitement contre la surchauffe et protéger la surface de la peau contre les brûlures; le fait que les conduites débouchent au voisinage du ballonnet permet de refroidir préférentiellement le ballonnet au voisinage de la peau. Le niveau du liquide dans le corps de sonde est choisi - dans l'exemple des figures 1 à 3 - de sorte que la hauteur de liquide et donc la pression dans le ballonnet soient constants lors du passage du mode imagerie en mode traitement. Cette contrainte n'existe pas dans l'exemple des figures 8 et 9.

Est en outre prévu un cache, dont la fonction est expliquée en référence à la figure 2. La figure montre encore le transducteur d'imagerie 18, ainsi que la platine 20 sur laquelle ce transducteur est monté en translation suivant l'axe longitudinal de la sonde. Le transducteur d'imagerie est par exemple une barrette linéaire ultrasonore, qui assure une grande précision d'imagerie. La partie arrière 22 du corps de sonde, à l'opposé de la partie frontale, contient les actionneurs de déplacement des transducteurs.

La figure 1 montre encore l'allure du faisceau d'ultrasons, qui présente, dans le plan de la direction longitudinale du transducteur de traitement, une forme conique; le sommet du cône 24 est le foyer, auquel sont concentrés les ultrasons. En traitement, le sommet recouvre la cible.

Comme le montre la figure, l'axe de rotation 8 du corps de sonde passe sensiblement par le foyer, le transducteur de traitement s'étend dans un plan qui est sensiblement perpendiculaire à l'axe de rotation 8; de la sorte, l'axe acoustique du transducteur est sensiblement confondu avec l'axe de rotation de la sonde. En pratique, il est avantageux que l'axe acoustique soit exactement confondu avec l'axe de rotation de la sonde - aux jeux mécaniques éventuels près. On peut néanmoins admettre un débattement entre l'axe acoustique et l'axe de rotation, dans la mesure où le déplacement du point focal lors de la rotation du corps de sonde autour de l'axe de rotation reste limité, et reste typiquement inférieur à la dimension transversale de la zone focale. En termes de distance, la distance entré l'axe acoustique et l'axe de la sonde est avantageusement inférieure à 1 mm, dans toute la zone s'étendant entre le foyer et le transducteur de traitement; en termes d'angles, on peut admettre un angle entre la direction de l'axe acoustique et la direction de l'axe de rotation qui va jusqu'à 15°.

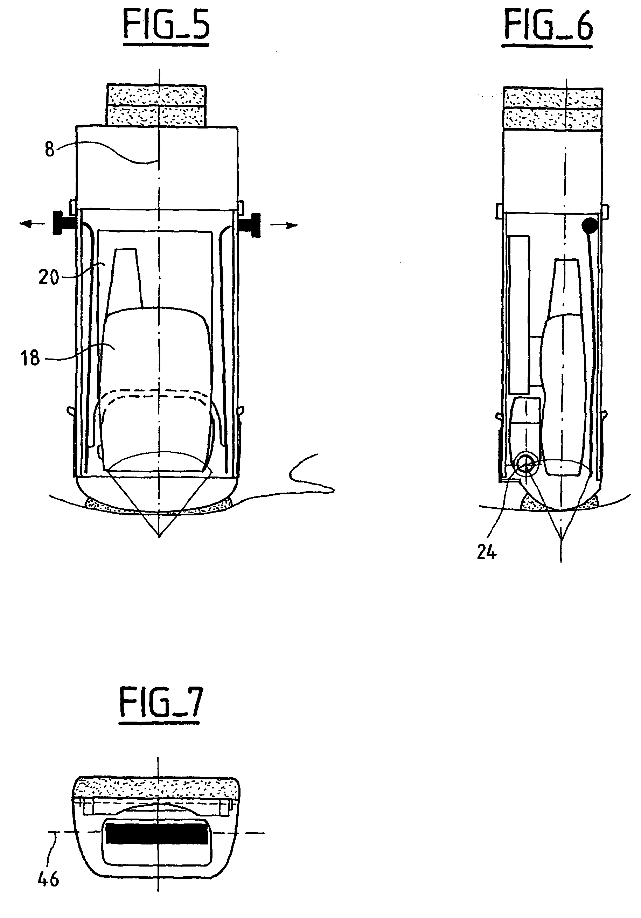

La figure 2 est une vue schématique en coupe longitudinale d'une sonde, suivant la direction transversale du transducteur de traitement. On reconnaît sur la figure les éléments déjà décrits en référence à la figure 1. La figure 2 montre encore que le transducteur de traitement est monté à rotation autour d'un arbre 24; la rotation du transducteur de traitement lui permet de s'escamoter pour laisser passer le transducteur d'imagerie lorsque la sonde est en position d'imagerie, comme représenté aux figures 5, 6 et 7. La figure 2 montre aussi la fonction du cache; du fait de la présence de la platine 20 et de l'arbre 24, le corps de sonde n'est pas symétrique par rapport au plan contenant l'axe de rotation et la direction longitudinale du transducteur de traitement. Le ballonnet entoure l'extrémité du corps de sonde; le cache 26 a pour fonction de limiter les déformations du ballonnet, au voisinage de l'arbre 24. Ainsi, le ballonnet s'appuie sur un contour rigide, défini par les bords du corps de sonde et par le cache; ce contour est symétrique ou sensiblement symétrique par rapport à l'axe de rotation ou par rapport à l'axe acoustique 8 de la sonde. Le ballonnet gonflé présente alors une forme symétrique par rapport à l'axe acoustique; notamment, comme le montrent les figures, le point le plus avant du ballonnet est sensiblement sur l'axe acoustique. Ceci favorise le positionnement du corps de sonde par rapport au patient. Lorsque le corps de sonde est entraîné en rotation autour de l'axe 8, ceci assure que le point de contact ou la zone de contact entre le ballonnet et le patient soit aussi sensiblement sur l'axe de rotation.

La figure 2 montre, comme la figure 1, l'allure du faisceau d'ultrasons, qui présente aussi dans le plan de la direction transversale du transducteur de traitement, une forme conique; le cône de la figure 2 est toutefois d'un angle au centre plus faible que l'angle au centre du cône de la figure 1. La différence d'angle est représentative de l'allongement du transducteur de traitement, autrement dit du rapport entre sa dimension longitudinale et sa dimension transversale. Dans l'exemple, le transducteur présente une dimension longitudinale de l'ordre de 54 mm, et une dimension transversale de l'ordre de 35 mm. Le rapport entre ces deux dimensions - l'allongement du transducteur de traitement - est de 1,6. En pratique, des valeurs d'allongement comprises entre 2,5 et 1,2 conviennent. La borne inférieure est déterminée par la volonté de disposer d'une direction privilégiée pour le traitement; la borne supérieure est fonction des limitations sur la taille des transducteurs et la capacité des ultrasons à traverser les tissus. La focale du transducteur dépend de l'application envisagée et de la profondeur de l'organe à traiter. Dans le cas de la thyroïde, une focale entre 30 et 45 mm est adaptée.

La figure 2 montre encore que l'axe de rotation 8 du corps de sonde passe sensiblement par le foyer du transducteur de traitement. Comme ceci a été expliqué plus haut en ce qui concerne l'axe acoustique, il est avantageux que l'axe de rotation soit exactement contenu dans le plan d'imagerie; des variations sont possibles, dans les mêmes plages que celles qui sont indiquées plus haut pour l'axe acoustique.

La figure 3 est une vue schématique de face du corps de sonde des figures 1 et 2. On y reconnaît le transducteur de traitement, le cache, ainsi que l'axe de l'arbre 24. La figure 3 montre bien que le contour sur lequel s'appuie le ballonnet est sensiblement symétrique par rapport à l'axe de rotation. La figure montre encore que le transducteur de traitement est un transducteur à pan coupés.

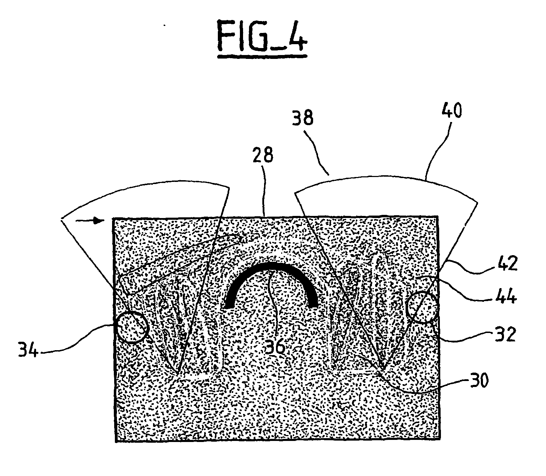

La figure 4 est une vue schématique de la sonde des figures 1 et 2, dans une application au traitement de la thyroïde; on a représenté à la figure une vue en coupe dans un plan horizontal; on y reconnaît la peau 28 du cou du patient, la thyroïde 30, les deux artères carotides 32 et 34, ainsi que la trachée 36. La thyroïde présente deux lobes qui s'étendent de part et d'autre de la trachée, et sont pratiquement au contact des artères carotides. La figure montre aussi la position du cône des ultrasons pour le traitement du lobe droit de la carotide; on a représenté le cône des ultrasons 38 ou 40 pour deux positions de la sonde; les zones focales 42 ou 44 sont aussi représentées pour ces deux positions de la sonde. La figure montre que la glande est peu profonde et donc facilement accessible aux ultrasons. Cependant, dans les plans transversaux (dans un repère lié au patient) la fenêtre acoustique entre la trachée et la partie externe du cou est relativement étroite. La forme allongée du transducteur, et l'utilisation du transducteur avec la direction longitudinale parallèle au cou du patient permet de disposer, dans la direction transversale, d'un cône plus fermé que dans la direction longitudinale. Ceci permet de conserver un traitement efficace - une surface donnée de transducteur de traitement - tout en préservant les organes voisins de la glande à traiter.

Ne sont pas représentés à la figure les nerfs récurrents, qui commandent les cordes vocales et passent en arrière des deux lobes de la glande thyroïdienne. Ces nerfs sont protégés lors du traitement, dans la mesure où ils se trouvent en arrière de la zone visée. N'est pas non plus représenté l'oesophage, qui se trouve derrière la trachée.

Pour renforcer la sécurité du traitement, il est aussi possible d'utiliser la sonde décrite avec une sonde trachéale, qui est installée dans la trachée du patient pendant les traitements. Par exemple la sonde peut être un Moniteur d'intégrité nerveuse NIM-Response®, commercialisée par la société Xomed. Elle comporte des électrodes placées à proximité des nerfs récurrents, qui permettent ainsi de détecter des altérations éventuelles à ces nerfs dues à l'énergie ultrasonore émise pendant le traitement.

Il est également possible d'utiliser une sonde trachéale comportant un ballonnet, positionné en regard de la thyroïde et parcouru par une circulation d'eau froide, ce qui permet de refroidir la trachée et donc de la protéger des dommages thermiques éventuels. Une sonde combinant des électrodes et une circulation froide peut être également utilisée.

Les figures 5, 6 et 7 sont des vues schématiques correspondant aux figures 1, 2 et 3, lorsque la sonde est en position d'imagerie. Dans cette position, le transducteur de traitement a basculé autour de l'arbre 24 pour se plaquer contre la paroi du corps de sonde; en revanche, la platine 20 fait avancer le transducteur d'imagerie 18, de sorte à ce qu'il vienne prendre la place libérée par le transducteur de traitement. Dans cette position d'imagerie, le plan d'imagerie - ou plan de balayage du transducteur d'imagerie - contient l'axe de rotation 8 du corps de sonde. De préférence la surface d'émission du transducteur d'imagerie est également perpendiculaire à l'axe de rotation 8 du corps de sonde. Ce plan est parallèle au plan de la figure 5 et est référencé 46 sur la figure 7. La présence de la platine permet d'approcher ou d'éloigner le transducteur d'imagerie du tissu, afin d'optimiser la qualité de l'image. En effet dans certains cas, des échos parasites se superposent à la zone focale, et la translation de la sonde permet de les déplacer. De plus certains échographes ont une focale fixe, et le déplacement du transducteur d'imagerie permet de situer la cible dans la plage dans la quelle l'image est la plus fine.

Dans la position d'imagerie de la sonde, le plan d'imagerie contient l'axe acoustique, et donc contient aussi l'axe de rotation du corps de sonde. Il est donc possible, lors de la rotation du corps de sonde, d'obtenir de façon continue l'image de la zone de l'organe à traiter qui est couverte par la cible. Il est aussi possible de marquer sur l'image échographique la position du foyer ou même la position et l'étendue des lésions qui seront effectuées par le transducteur de traitement.

Pour revenir dans la position de traitement représentée aux figures 1 à 3, le transducteur d'imagerie est retiré vers l'arrière du corps de sonde, et le transducteur de traitement est rabattu en place, de sorte que son axe acoustique soit à nouveau confondu avec l'axe de rotation du corps de sonde. Escamoter le transducteur d'imagerie pendant le traitement permet de disposer d'une surface émissive maximale pour le transducteur de traitement.

Les figures 8 et 9 sont des vues schématiques partielles d'un autre exemple de sonde, en coupe longitudinale suivant la direction longitudinale du transducteur de traitement et en vue de face. L'exemple des figures 8 et 9 diffère de l'exemple des figures précédentes en ce que le transducteur d'imagerie est intégré au transducteur de traitement; dans l'exemple, le transducteur d'imagerie est placé dans une ouverture pratiquée dans le transducteur de traitement. Ceci rend inutile tout déplacement du transducteur d'imagerie ou du transducteur de traitement; en conséquence, le transducteur de traitement et d'imagerie peut être dans une position fixe à l'intérieur du corps de sonde. Celui-ci peut présenter une forme symétrique par rapport à l'axe de rotation, et de ce fait, le cache n'est pas nécessaire.

La figure 8 montre donc la paroi 48 du corps de sonde, le ballonnet 50 disposé à son extrémité frontale, le transducteur 52 avec la partie 54 servant pour l'imagerie et la partie 56 servant pour le traitement.. On a porté sur les figures, comme précédemment, le cône 58 formé par les ultrasons, l'axe de rotation 60 et la peau 62 du patient. Comme dans l'exemple précédent, le transducteur de traitement présente une forme allongée et son axe acoustique est sensiblement confondu avec l'axe de rotation du corps de sonde.

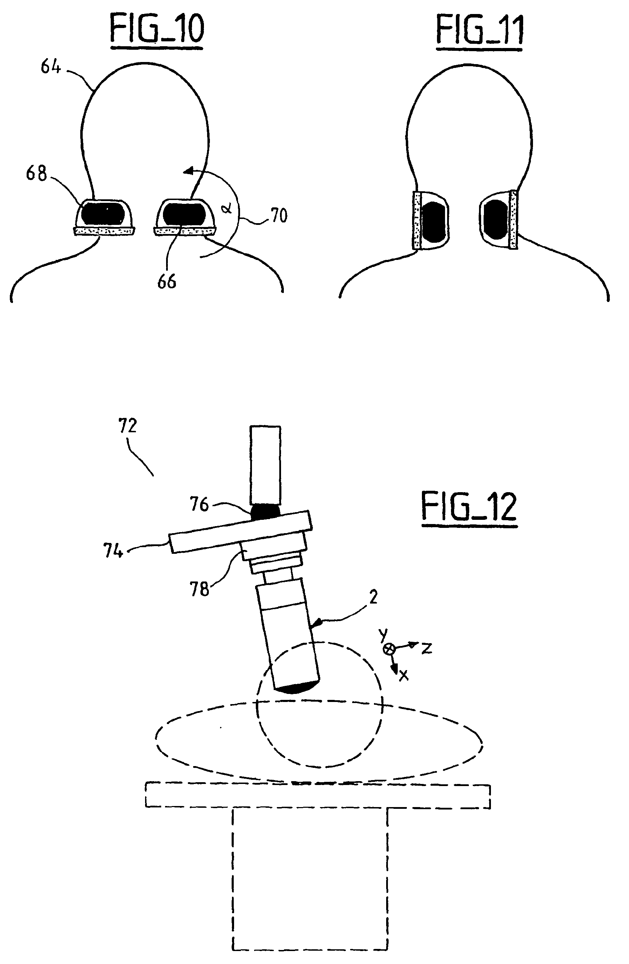

Les figures 10 et 11 sont des vues de la position du corps de sonde par rapport à un patient, dans une application au traitement de la thyroïde; elles montrent schématiquement le patient 64, ainsi qu'une sonde 66 ou 68, en position au voisinage du lobe gauche ou du lobe droit de la thyroïde. On appelle pour le patient la direction longitudinale la direction allant de la tête au pieds du patient, et direction transversale la direction allant d'un lobe de la thyroïde à l'autre lobe.

La figure 10 montre une position d'imagerie du corps de sonde. Dans cette position, le patient est allongé et la sonde est amenée au contact de la peau du cou du patient, en face du lobe concerné de la thyroïde. L'axe de rotation de la sonde est alors dans un plan vertical, perpendiculaire à la direction transversale du patient. Comme le symbolise la flèche 70 sur la figure 10, il est possible d'entraîner en rotation le corps de sonde, autour de l'axe de rotation; ceci permet de faire varier le plan d'imagerie du transducteur d'imagerie.

La figure 11 montre une position de traitement du corps de sonde. Dans cette position, l'axe longitudinal du transducteur de traitement est parallèle à l'axe longitudinal du patient. La forme allongée du transducteur de traitement permet un traitement plus précis, sans risquer d'endommager les organes voisins des lobes de la thyroïde.

La figure 12 est une vue schématique d'un exemple de sonde, montrant le support du corps de sonde; Celui-ci comprend une potence 72, sur laquelle est montée une platine de translation 74, à l'aide d'une rotule 76. Une deuxième platine de translation 78 est montée sur la première platine, avec une direction de déplacement perpendiculaire à celle de la première platine. Le corps de sonde est monté en rotation sur la deuxième platine 78, avec un axe de rotation perpendiculaire aux directions de déplacement des deux platines. Les directions x, y et z de l'axe de rotation, de la direction de déplacement de la deuxième platine et de la direction de déplacement de la première platine forment ainsi un repère orthonormé.

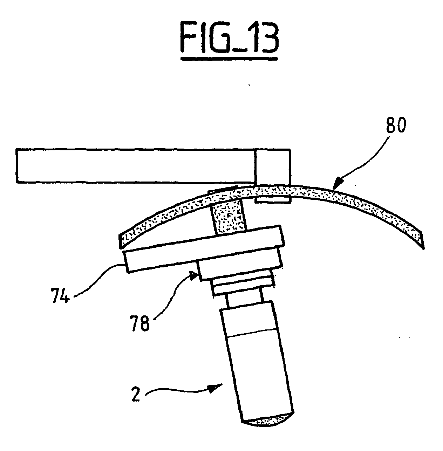

La figure 13 est une vue analogue à celle de la figure 12, pour un autre exemple de support de sonde ; le support de sonde de la figure 13 diffère de celui de la figure 12 en ce que la première platine 74 est montée sur la potence par l'intermédiaire d'un arceau 80, le long duquel la première platine peut se déplacer. L'arceau 80 est lui-même monté à rotation sur la potence selon un axe vertical, ce qui permet d'incliner la sonde dans toutes les directions. Il est avantageux que le rayon de l'arceau soit tel que le déplacement de la première platine 74 le long de l'arceau s'effectue autour du foyer. Autrement dit, le rayon de l'arceau est sensiblement égal à la distance entre l'arceau et le foyer.

Dans un cas comme dans l'autre, les deux platines permettent un déplacement de la sonde, perpendiculairement à l'axe acoustique. En effet, chaque impulsion acoustique provoque la nécrose du tissu dans un petit volume. Pour traiter une cible complète, on déplace donc la tête entre les tirs, grâce aux platines.

Il est aussi avantageux d'orienter la partie frontale de la sonde sensiblement perpendiculaire à la peau. Ceci permet de conserver une profondeur constante d'un déplacement à l'autre de la tête. L'orientation est possible grâce à la rotule de la figure 12 ou à l'arceau de la figure 13. L'un comme l'autre assure la profondeur constante.

La sonde décrite présente les avantages suivants. Elle assure d'abord un repérage précis. En effet, le transducteur de traitement est associé à un transducteur d'imagerie, procurant une image de grande finesse et capable de visualiser toute la cible. Dans la mesure où les positions relatives du transducteur de traitement et du transducteur d'imagerie peuvent être déterminées avec précision - dans un exemple comme dans l'autre - les images obtenues grâce au transducteur d'imagerie sont en relation spatiale connue et précise avec la zone focale du transducteur de thérapie; l'effet des ultrasons se produit bien dans la zone visualisée par le transducteur d'imagerie. La rotation du corps de sonde autour d'un axe qui est confondu avec l'axe acoustique du transducteur de traitement et qui est contenu dans le plan d'imagerie assure une bonne précision, même lors des déplacements de la sonde.

La sonde assure aussi un traitement sûr, notamment dans le cas des traitements de la thyroïde. Les nerfs récurrents sont protégés par les deux lobes de la glande thyroïdienne. Le positionnement en profondeur du point focal, par un gonflage approprié du ballonnet, assure une grande précision du traitement en profondeur. La trachée est protégée par le positionnement du corps de sonde. L'oesophage est situé derrière la trachée et protégé par celle-ci. Le débit sanguin très important au sein de la carotide la protège des effets thermiques des ultrasons. Comme expliqué plus haut, la forme allongée du transducteur de traitement permet de préserver les tissus, en utilisant un faisceau ultrasonore en forme de cône aplati.

La sonde assure aussi un traitement efficace. Pour obtenir un effet de traitement, par exemple par coagulation du tissu dans la zone focale, les ondes ultrasonores doivent être suffisamment concentrées. Pour cela, on admet généralement que le diamètre du transducteur est environ égal à sa focale. De plus la puissance est fonction de la surface émissive. La forme allongée du transducteur permet de satisfaire la contrainte sur le diamètre du transducteur, sans réduire la surface émissive.

La sonde est aussi d'une utilisation simple. En effet, elle s'adapte facilement possible à tous les patients : l'empreinte de la sonde - la projection sur le patient suivant l'axe acoustique, ou la zone de contact de la sonde avec le patient est minimale, et correspond sensiblement à la taille du transducteur de thérapie.

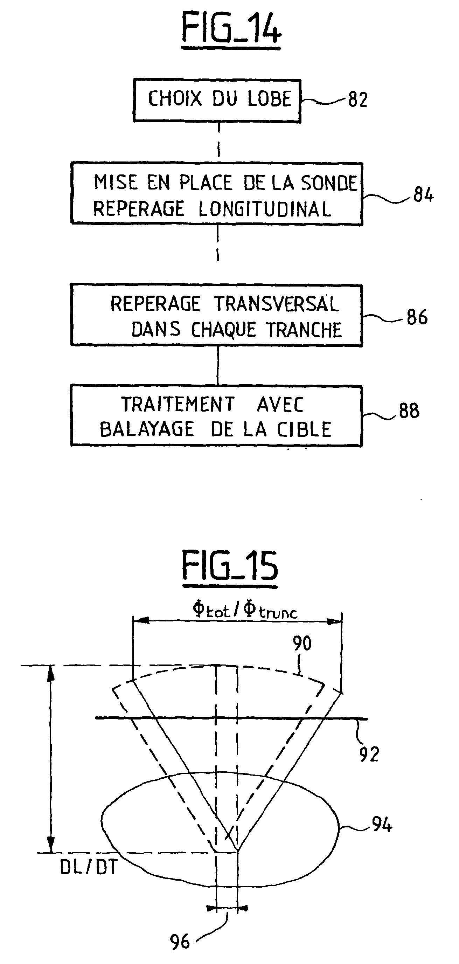

Un exemple de séquence de fonctionnement de la sonde est maintenant décrit, en référence à la figure 14. Il est fait référence à un écran, qui peut indiquer à l'opérateur les consignes à suivre et lui permettre de visualiser les images obtenues par le transducteur d'imagerie, ainsi qu'à des moyens d'entrée des limites de la cible ; ces moyens d'entrée peuvent typiquement comprendre un dispositif de pointage d'une nature quelconque. L'ensemble du traitement est alors piloté par ordinateur ; l'utilisation d'un ordinateur ou d'un matériel analogue pour la commande d'un appareil de traitement par ultrasons est connue en soi et n'est pas expliquée en détail.

A l'étape 82, l'opérateur commence par indiquer quel côté du patient est traité - droit ou gauche. L'orientation longitudinale de la sonde dépend du côté à traiter, comme le montre la figure 11 . La sonde est ensuite commutée en mode repérage ou imagerie. Ceci peut consister à passer de la position de la figure 1 à celle de la figure 5. Alternativement, dans le cas de la sonde de la figure 8, il suffit de commuter le générateur d'impulsion des transducteurs d'imagerie et de traitement.

A l'étape 84, la sonde est appliquée sur le patient et orientée dans l'axe longitudinal du patient. A cette étape, le transducteur, son faisceau acoustique et la future lésion peuvent être symbolisés à l'écran. Le praticien marque sur l'image les positions extrêmes « tête » et « pieds », qui correspondent à l'extension céphalo-caudale de la cible. Ce repérage définit une série de « tranches » ou plans de traitement successifs, qui sont orientés transversalement par rapport au patient. Le praticien marque également la position de la peau, pour calcul ultérieur de la puissance à délivrer lors des tirs. Cette étape permet de déterminer les positions successives de la sonde, suivant la direction longitudinale du patient. La distance entre les "tranches" est fonction de la taille de la zone entourant le foyer dans laquelle les tissus sont traités. Une distance entre 10 et 30 mm est appropriée.

A l'étape 86, la sonde est ensuite déplacée vers la première tranche, qui est la position marquée « tête ». La sonde peut alors être orientée dans l'axe transversal du patient. A cet effet, le transducteur, son faisceau acoustique tel qu'il sera lors du traitement, c'est à dire lorsque la sonde sera orientée longitudinalement, et la future lésion sont symbolisés à l'écran. L'opérateur peut repérer les lésions à traiter dans la première tranche.

L'opérateur déplace ensuite la sonde vers la deuxième tranche, dans la direction caudale. Il procède de la même façon que pour la première tranche au repérage de la peau et des lésions à traiter dans cette deuxième tranche. La sonde est ensuite déplacée vers les tranches suivants, en procédant à chaque fois au repérage.

A l'issue de ces étapes 82 à 86 , les contours de la cible sont déterminés, et on connaît dans chaque tranche les limites choisies par l'opérateur pour le traitement par ultrasons. On peut bien entendu procéder autrement, par exemple inverser les directions de balayage et utiliser des "tranches" verticales". Toutefois le marquage des contours de la cible sur les tranches horizontales permet d'éviter plus facilement les structures à préserver, telles que la trachée ou les nerfs parce que celles-ci sont plus facilement visibles sur des coupes transversales du cou. Au cours des étapes 84 et 86, il est possible de faire tourner la sonde, pour faire varier la direction du plan d'imagerie, comme expliqué en détail plus haut.

A l'étape 88, la sonde est ensuite commutée en mode tirs; ceci peut consister à passer de la position de la figure 5 à celle de la figure 1. Alternativement, dans le cas de la sonde de la figure 8, il suffit de commuter à nouveau le générateur d'impulsions des transducteurs; la sonde est positionnée en rotation de sorte que l'axe longitudinal du transducteur de traitement soit confondu avec l'axe longitudinal du patient. On procède ensuite au traitement, en balayant la cible suivant les paramètres de position définis aux étapes 84 et 86. Des indications sur les paramètres de traitement sont données plus bas. De préférence, et parce qu'il y a toujours un risque de mouvement du patient, les premiers tirs ont lieu à proximité des structures à préserver. Dans le cas du traitement de la thyroïde, on préfère traiter d'abord le plan interne; ceci revient à supposer que la position du patient au début d'une séquence de traitement est correcte, et que si elle évolue ce n'est qu'ensuite. Dans le sens vertical, on préférera traiter d'abord les tranches caudales.

On donne maintenant à titre d'exemple des paramètres de traitement possible, dans le cas de la thyroïde. La figure 15 montre une vue du transducteur de traitement avec les notations utilisées. On a porté à la figure une représentation schématique du transducteur de traitement 90, de la peau 92 et de la cible 94. La figure montre la distance focale - distance entre le transducteur de traitement et le foyer mesurée sur l'axe acoustique 96. Elle montre aussi la distance entre le transducteur de traitement et la peau, mesurée aussi le long de l'axe acoustique. La figure montre en traits continus une position du transducteur de traitement, et en traits interrompus une autre position du transducteur de traitement.

On utilise les notations suivantes :

- f fréquence d'excitation du transducteur
- $P_{ref\,E}$ puissance de référence du transducteur en W : c'est la puissance électrique à fournir pour que le transducteur engendre l'intensité requise au foyer pour obtenir une nécrose de coagulation des tissus dans la zone focale ;
- η rendement électro-acoustique du transducteur: rapport entre la puissance acoustique fournie par le transducteur et la puissance électrique appliquée ;
- $P_{ref\,A}$ puissance de référence du transducteur en W : c'est la puissance acoustique que doit fournir le transducteur pour obtenir une nécrose de coagulation des tissus dans la zone focale; elle est définie par $P_{ref\,A} = P_{ref\,E}{}^{*}\,\eta$
- $D_{foc}$ distance focale du transducteur. Les focales courtes seront préférables pour les patients maigres ;
- $\Phi_{tot}$ diamètre du transducteur, ou dimension du transducteur selon sa direction longitudinale ;
- $\Phi_{trunc}$ diamètre coupé du transducteur ou dimension du transducteur selon sa direction transversale ;

Les deux derniers paramètres peuvent s'exprimer en fonction de "l'ouverture" qui est le rapport entre le grand diamètre et la distance focale: $N=\Phi_{tot}\,/\,D_{foc}$ et le paramètre de troncature $R=\Phi_{trunc}\,/\,\Phi_{tot}$, qui est l'inverse de l'allongement discuté plus haut.

On note encore :

- $D_L$ espacement entre les points dans la direction longitudinale ;
- $D_T$ espacement entre les points dans la direction transversale ;

- $T_{on}$ durée de chaque impulsion ;
- $T_{off}$ attente entre chaque impulsion.

La puissance peut être calculée en compensant l'absorption des tissus par une augmentation de la puissance suivant la formule :

$$P = P_{ref} * \exp(2*\alpha*f*D_{ep}/10)$$

où:

$\alpha$ coefficient d'absorption des tissus en Np/cm/MHz. Pour les traitements HIFU, on utilisera une valeur supérieure (0,06 à 0,08) à celles données par diverses sources bibliographiques et qui sont utilisables en échographie (typiquement 0,04 à 0,05).
$D_{ep}$ épaisseur des tissus traversés en mm
$D_{ep}$ est fonction de la distance à la peau et de la focale,
$D_{ep} = D_{foc} - S$ avec :
$S$ Distance du transducteur à la peau

Il a été montré expérimentalement que les paramètres suivants sont particulièrement bien adaptés au traitement des nodules thyroïdiens chez l'homme. On note minn et maxx les valeurs minimales et maximales admises, min et max les bornes des plages conseillées de valeur et Typ une valeur typique considérée à titre d'exemple.

|  |  | minn | Min | Typ | max | maxx |  |
|---|---|---|---|---|---|---|---|
| Pref A | W | 5 | 9 | 11 | 13 | 18 | Puissance de référence du transducteur |
| $\alpha$ | Np/cm/MHz | 0,05 | 0,06 | 0,07 | 0,08 | 0,1 | Coefficient d'absorption des tissus |
| F | MHz | 2 | 2,5 | 3 | 3,5 | 4,5 | Fréquence d'excitation du transducteur |
| Dfoc | mm | 25 | 30 | 40 | 45 | 50 | Distance focale du transducteur |
| S | mm | 10 | 12 | 15 | 25 | 40 | Distance du transducteur à la peau |
| N |  | 1,00 | 1,04 | 1,25 | 1,36 | 1,43 | ouverture du transducteur |
| R |  | 51% | 61% | 70% | 76% | 100% | troncature du transducteur |
| DL | mm |  | 1,6 | 1,7 | 1,8 |  | Espacement des points dans la direction longitudinale |
| DT | mm |  | 1,6 | 1,7 | 1,8 |  | Espacement des points dans la direction transversale |
| Ton | s | 2 | 2,5 | 3 | 3,5 | 6 | durée de chaque impulsion |
| Toff | s | 3 | 5 | 10 | 15 | 20 | attente entre chaque impulsion |

Ces valeurs permettent un traitement en une séance d'une durée unitaire / totale de 15 minutes environ. On constate, dans un délai de plusieurs semaines après le traitement, la disparition des nodules, le tissu traité étant remplacé par une fibrose.
Bien entendu, la présente invention n'est pas limitée aux exemples et modes de réalisation décrits-et représentés, mais elle est susceptible de nombreuses variantes accessibles à l'homme de l'art. On pourrait ainsi utiliser d'autres formes allongées de transducteurs de traitement. La cinématique du transducteur d'imagerie et du transducteur de traitement peut être différente de celle mentionnée aux figures 1 à 7. Ainsi, on pourrait prévoir un troisième déplacement pour la sonde, parallèlement à l'axe acoustique, si le praticien désire éloigner ou rapprocher la sonde du tissu. La rotule de la figure 12 peut être remplacée par deux axes perpendiculaires.
Il est aussi clair que la sonde n'est pas limitée à l'application préférée de traitement de la thyroïde; elle peut aussi être utilisée pour le traitement d'autres organes, comme par exemple les tumeurs de la région du cou, les tumeurs du sein, les tumeurs osseuses ou tout autre organe ou anomalie tissulaire accessibles aux ultrasons par voie extracorporelle. Les paramètres de tir fournis dans le tableau peuvent être modifiés, en fonction de l'organe à traiter, de l'effet thérapeutique recherché, des caractéristiques du transducteur, etc.

**Revendications**

1. Une sonde de traitement par ultrasons focalisés, comportant :

   - un corps de sonde (2) monté à rotation autour d'un axe (8);
   - un transducteur de traitement (10) de forme allongée, avec un axe acoustique d'émission d'ultrasons focalisés sensiblement confondu avec l'axe de rotation du corps de sonde et procurant un faisceau ultrasonore en forme de cône dissymétrique présentant un angle au sommet moins ouvert dans une première direction que dans une direction transversale à la première direction;
   - un transducteur d'imagerie (18) dont le plan d'imagerie contient l'axe acoustique du transducteur de traitement.

2. La sonde de la revendication 1, **caractérisée en ce que** le transducteur de traitement (10) est monté mobile dans le corps de sonde (2).

3. La sonde de la revendication 2, **caractérisée en ce que** le transducteur de traitement est monté à rotation dans le corps de sonde, de préférence autour d'un axe perpendiculaire à l'axe de rotation (8).

4. La sonde selon l'une quelconque des revendications 1 à 3, **caractérisée en ce que** le transducteur d'imagerie (18) est monté mobile dans le corps de sonde (2).

5. La sonde de la revendication 4, **caractérisée en ce que** le transducteur d'imagerie est monté en translation dans le corps de sonde, de préférence suivant une direction parallèle à l'axe de rotation (8).

6. La sonde de l'une des revendications 1 à 5, **caractérisée en ce que** le transducteur de traitement (10) présente un allongement supérieur à 1,2.

7. La sonde de l'une des revendications 1 à 6, **caractérisée en ce que** le transducteur de traitement (10) présente un allongement inférieur à 2,5.

8. La sonde de l'une des revendications 1 à 7, **caractérisée en ce que** le transducteur d'imagerie est une barrette effectuant un balayage linéaire.

9. La sonde de la revendication 8, **caractérisée en ce que** l'axe du transducteur d'imagerie est parallèle à la direction longitudinale du transducteur de traitement.

10. La sonde de l'une des revendications 1 et 6 à 9, **caractérisée en ce que** le transducteur d'imagerie est intégré au transducteur de traitement.

11. La sonde de l'une des revendications 1 à 10, **caractérisée en ce qu'**elle présente un support sur lequel la sonde est montée à rotation, le support (74, 78) déplaçant la sonde en translation dans un plan perpendiculaire à l'axe de rotation (8).

12. La sonde de la revendication 11, **caractérisé en ce que** le support déplace en outre la sonde en translation suivant la direction de l'axe de rotation.

13. La sonde de la revendication 11 ou 12, **caractérisée en ce que** le support présente une rotule (76) d'orientation de la sonde.

14. La sonde de la revendication 11 ou 12, **caractérisée en ce que** le support présente un arceau (80) le long duquel la sonde se déplace.

15. La sonde de la revendication 14, **caractérisée en ce que** le rayon de l'arceau est sensiblement égal à la distance entre l'arceau et le foyer du transducteur de traitement.

16. La sonde selon l'une quelconque des revendications 1 à 15, dans laquelle :

- l'extrémité du corps de sonde (2) est formée par un ballonet (12, 50) souple et transparent aux ultrasons, le ballonet étant adapté à être gonflé par un liquide de couplage transparent aux ultrasons ; et
- le ballonnet s'appuie sur un contour rigide symétrique par rapport à l'axe acoustique (8) du transducteur de traitement (10), le point le plus en avant du ballonnet gonflé étant sensiblement sur l'axe acoustique (8).

17. La sonde selon la revendication 16, dans laquelle le ballonet gonflé présente une forme symétrique par rapport à l'axe acoustique (8).

18. La sonde selon la revendication 17, dans laquelle :

    - le corps de sonde (2) n'est pas symétrique par rapport au plan contenant l'axe de rotation du corps de sonde et la direction longitudinale du transducteur de traitement ; et
    - le contour rigide sur lequel s'appuie le ballonet est défini par les bords du corps de sonde et par un cache (26).

19. Un appareil de traitement par ultrasons, comprenant :

    - une sonde de traitement par ultrasons focalisés selon l'une quelconque des revendications 1 à 18 ;
    - un écran permettant de visualiser les images obtenues par le transducteur d'imagerie de la sonde ;
    - des moyens permettant de symboliser à l'écran le transducteur de traitement de la sonde, le faisceau acoustique du transducteur de traitement de la sonde et la cible à traiter ;
    - des moyens d'entrée des limites de la cible ; et
    - un ordinateur de pilotage du traitement.

20. L'appareil de traitement par ultrasons selon la revendication 19, dans lequel lesdits moyens sont adaptés pour symboliser à l'écran ledit faisceau acoustique tel qu'il sera lors du traitement, à un moment où la sonde de traitement a une orientation transversale à celle lors du traitement.

**Patentansprüche**

1. Eine Sonde zur Behandlung durch fokussierten Ultraschall, welche umfasst:

    - einen Sondenkörper (2), rotationsbeweglich um eine Achse (8) angebracht;
    - einen Behandlungswandler (10) in langgestreckter Form, mit einer akustischen Achse zur Aussendung von fokussiertem Ultraschall, welche deutlich mit der Rotationsachse des Sondenkörpers zusammenfällt, und einen Ultraschallstrahl in Form eines unsymmetrischen Kegels erzeugt, welcher einen in einer ersten Richtung weniger als in einer Querrichtung zur ersten Richtung geöffneten Scheitelwinkel aufweist;
    - einen Bildwandler (18), dessen Bildebene die akustische Achse des Behandlungswandlers enthält.

2. Sonde nach Anspruch 1, **dadurch gekennzeichnet, dass** der Behandlungswandler (10) beweglich im Sondenkörper (2) angebracht ist.

3. Sonde nach Anspruch 2, **dadurch gekennzeichnet, dass** der Behandlungswandler rotationsbeweglich im Sondenkörper angebracht ist, vorzugsweise um eine zur Rotationsachse senkrechten Achse (8).

4. Sonde nach einem beliebigen der Ansprüche 1 bis 3, **dadurch gekennzeichnet, dass** der Bildwandler (18) beweglich im Sondenkörper (2) angebracht ist.

5. Sonde nach Anspruch 4, **dadurch gekennzeichnet, dass** der Bildwandler im Sondenkörper translatorisch angebracht ist, vorzugsweise in einer zur Rotationsachse parallelen Richtung (8).

6. Sonde nach einem der Ansprüche 1 bis 5, **dadurch gekennzeichnet, dass** der Behandlungswandler (10) eine Streckung größer als 1,2 darstellt.

7. Sonde nach einem der Ansprüche 1 bis 6, **dadurch gekennzeichnet, dass** der Behandlungswandler (10) eine Streckung kleiner als 2,5 darstellt.

8. Sonde nach einem der Ansprüche 1 bis 7, **dadurch gekennzeichnet, dass** der Bildwandler ein Sensor ist, welcher ein lineares Abtasten ausführt.

9. Sonde nach Anspruch 8, **dadurch gekennzeichnet, dass** die Achse des Bildwandlers parallel zur Längsrichtung des Behandlungswandlers ist.

10. Sonde nach einem der Ansprüche 1 und 6 bis 9, **dadurch gekennzeichnet, dass** der Bildwandler im Behandlungswandler integriert ist.

11. Sonde nach einem der Ansprüche 1 bis 10, **dadurch gekennzeichnet, dass** sie einen Träger aufweist, auf welchem die Sonde rotationsbeweglich angebracht ist, wobei der Träger (74, 78) die Sonde translatorisch in einer zur Rotationsachse (8) senkrechten Ebene bewegt.

12. Sonde nach Anspruch 11, **dadurch gekennzeichnet, dass** der Träger zusätzlich die Sonde translatorisch in Richtung der Rotationsachse bewegt.

13. Sonde nach Anspruch 11 oder 12, **dadurch gekennzeichnet, dass** der Träger ein Kugelgelenk (76) zur Ausrichtung der Sonde aufweist.

14. Sonde nach Anspruch 11 oder 12, **dadurch gekennzeichnet, dass** der Träger einen Bogen (80) aufweist, deren Länge nach sich die Sonde bewegt.

15. Sonde nach Anspruch 14, **dadurch gekennzeichnet, dass** der Radius des Bogen deutlich gleich dem Abstand zwischen dem Bogen und dem Fokus des Behandlungswandlers ist.

16. Sonde nach einem beliebigen der Ansprüche 1 bis 15, in welcher:

    - das Endstück des Sondenkörpers (2) durch einen für Ultraschall flexiblen und durchsichtigen Ballon (12, 50) geformt ist, wobei der Ballon geeignet ist, durch eine für den Ultraschall durchsichtige Flüssigkeit der Koppelung aufgeblasen zu werden; und
    - der Ballon sich an eine steife, im Bezug zur akustischen Achse (8) des Behandlungswandlers (10) symmetrische Kontur lehnt, wobei der vorderste Punkt des aufgeblasenen Ballons deutlich auf der akustischen Achse ist.

17. Sonde nach Anspruch 16, in welcher der aufgeblasene Ballon eine symmetrische Form im Bezug zur akustischen Achse (8) aufweist.

18. Sonde nach Anspruch 17, in welcher:

    - der Sondenkörper (2) nicht symmetrisch ist im Bezug zur Ebene, welche die Rotationsachse des Sondenkörpers und die Längsrichtung des Behandlungswandlers enthält; und
    - die steife Kontur, an welche sich der Ballon lehnt, durch die Ränder des Sondenkörpers und durch eine Abdeckung definiert ist (26).

19. Ein Ultraschallbehandlungsgerät, welches umfasst:

    - eine Sonde zur Behandlung durch fokussierten Ultraschall nach einem der Ansprüche 1 bis 18;
    - einen Bildschirm, welcher die durch den Bildwandler der Sonde erhaltenen Bilder zu visualisieren ermöglicht;
    - Mittel, die den Behandlungswandler der Sonde, den akustischen Strahl des Behandlungswandlers der Sonde und die Zielscheibe zum Behandeln am Bildschirm zu symbolisieren ermöglichen;
    - Mittel zu Eingabe der Grenzen der Zielscheibe; und
    - einen Computer zur Steuerung der Behandlung.

20. Ultraschallbehandlungsgerät nach Anspruch 19, in welchem die besagten Mittel geeignet sind, um am besagten Bildschirm den akustischen Strahl so zu symbolisieren, wie er während der Behandlung sein wird, in einem Moment, in welchem die Behandlungssonde eine Querausrichtung zu derjenigen während der Behandlung hat.

**Claims**

1. A treatment probe for focused ultrasound, comprising:

   - a probe body (2) mounted in rotation about an axis (8);
   - a treatment transducer (10) elongate in shape, with an acoustic axis for emission of focused ultrasound substantially the same as the axis of rotation of the probe body, forming an acoustic beam with the shape of an asymmetrical cone with an angle at the apex which is less open in a first direction than in a direction transverse to this first direction;
   - an imaging transducer (18) the imaging plane of which contains the acoustic axis of the treatment transducer.

2. The probe according to claim 1, **characterized in that** the treatment transducer (10) is mounted so that it is mobile in the probe body (2).

3. The probe according to claim 2, **characterized in that** the treatment transducer is mounted in rotation in the probe body, preferably about an axis perpendicular to the axis of rotation (8).

4. The probe according to one of claims 1 to 3, **characterized in that** the imaging transducer (18) is mounted so that it is mobile in the probe body (2).

5. The probe according to claim 4, **characterized in that** the imaging transducer is mounted in translation in the probe body, preferably following a direction parallel to the axis of rotation (8).

6. The probe according to one of claims 1 to 5, **characterized in that** the treatment transducer (10) has an aspect ratio of its dimensions greater than 1.2.

7. The probe according to one of claims 1 to 7, **characterized in that** the treatment transducer has an aspect ratio of its dimensions smaller than 2.5.

8. The probe according to one of claims 1 to 7, **characterized in that** the imaging transducer is an array carrying out a linear scan.

9. The probe according to claim 8, **characterized in that** the axis of the imaging transducer is parallel to the longitudinal direction of the treatment transducer.

10. The probe according to one of claims 1 and 6 to 9, **characterized in that** the imaging transducer is integrated in the treatment transducer.

11. The probe according to one of claims 1 to 10, **characterized in that** it has a support on which the probe is mounted in rotation, the support (74, 78) displacing the probe in translation in a plane perpendicular to the axis of rotation (8).

12. The probe according to claim 11, **characterized in that** the support moreover displaces the probe in translation following the direction of the axis of rotation.

13. The probe according to claim 11 or 12, **characterized in that** the support has a ball-and-socket joint (76) for orientation of the probe.

14. The probe according to claim 11 or 12, **characterized in that** the support has an arch (80) along which the probe moves.

15. The probe according to claim 14, **characterized in that** the radius of the arch is substantially equal to the distance between the arch and the focus of the treatment transducer.

16. The probe according to one of claims 1 to 15, in which :

    - the end of the body of the probe (2) is formed by a balloon (12, 50) that is flexible and transparent to ultrasound, the balloon being adapted to be inflated by an ultrasound-transparent coupling fluid, and
    - the balloon rests on a rigid contour symmetrical with respect to the acoustic axis (8) of the treatment transducer (10), the most forward point of the inflated balloon being substantially on the acoustic axis (8).

17. The probe according to claim 16, in a witch in the inflated balloon has a shape that is symmetrical with respect to the acoustic axis (8).

18. The probe according to claim 17, in which:

   - the body of the probe (2) is not symmetrical with respect to the plane containing the axis of rotation of the probe body and the longitudinal direction of the treatment transducer; and
   - the rigid contour against which the balloon bears is defined by the edges of the probe body and by a cover (26).

19. Ultrasound treatment apparatus comprising:

   - a probe for treatment by focused ultrasound according to any one of claims 1 to 18;
   - a screen allowing images obtained by the imaging transducer of the probe to be viewed;
   - means making it possible to symbolise, on the screen, the treatment transducer of the probe, and the acoustic beam of the treatment transducer of the probe, and the target to be treated;
   - means for entering the limits of the target; and
   - a computer for controlling treatment.

20. The ultrasound treatment apparatus according to claim 19, in which said means are adapted to symbolise on the screen said acoustic beam as it will be during treatment, at a point in time when the treatment probe has an orientation transverse to that at the time of treatment.

## FIG_1

## FIG_2

## FIG_3

15

# FIG_4

## FIG_5

## FIG_6

## FIG_7

## FIG_8

## FIG_9

## FIG_10

64

68

α

70

66

## FIG_11

## FIG_12

72

76

74

78

2

y

z

x

# FIG_13

## FIG_14

```
┌─────────────────┐
│  CHOIX DU LOBE  │──── 82
└─────────────────┘
         │
┌──────────────────────────┐
│ MISE EN PLACE DE LA SONDE│──── 84
│   REPERAGE LONGITUDINAL  │
└──────────────────────────┘
         │
┌──────────────────────────┐
│   REPERAGE TRANSVERSAL   │──── 86
│   DANS CHAQUE TRANCHE    │
└──────────────────────────┘
         │
┌──────────────────────────┐
│    TRAITEMENT AVEC       │──── 88
│  BALAYAGE DE LA CIBLE    │
└──────────────────────────┘
```

## FIG_15

$\Phi_{tot} / \Phi_{trunc}$

90

92

94

DL / DT

96